(19) Europäisches Patentamt
European Patent Office
Office européen des brevets

(11) Publication number : **0 187 009 B1**

(12) # EUROPEAN PATENT SPECIFICATION

(45) Date of publication of patent specification : **06.07.88**

(51) Int. Cl.⁴ : **C 07 C103/60**, A 61 K 31/16

(21) Application number : **85309141.1**

(22) Date of filing : **16.12.85**

(54) **Compounds and compositions having anti-inflammatory and analgesic activity.**

(30) Priority : **20.12.84 US 684427**

(43) Date of publication of application : **09.07.86 Bulletin 86/28**

(45) Publication of the grant of the patent : **06.07.88 Bulletin 88/27**

(84) Designated contracting states : **AT BE CH DE FR IT LI LU NL SE**

(56) References cited : **EP-A- 0 132 346**

(73) Proprietor : **THE PROCTER & GAMBLE COMPANY**
**One Procter & Gamble Plaza**
**Cincinnati Ohio 45202 (US)**

(72) Inventor : **Janusz, John Michael**
**2068 John Gray Road**
**Fairfield Ohio 45014 (US)**
Inventor : **Loomans, Maurice Edward**
**5231 Jessup Road**
**Cincinnati Ohio 45239 (US)**
Inventor : **Lahann, Thomas Robert**
**S.W. 850 Crestview**
**Pullman Washington 99163 (US)**

(74) Representative : **Brooks, Maxim Courtney et al**
**Procter & Gamble (NTC) Limited Whitley Road**
**Longbenton**
**Newcastle-upon-Tyne NE12 9TS (GB)**

**EP 0 187 009 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

## Description

### Technical field

The present invention relates to certain trienamides and pharmaceutical compositions containing these compounds which exhibit anti-inflammatory and analgesic activity.

### Background of the invention

Inflammation, or the « inflammatory response », is the result of complex interconnected physiological events, including increased vascular permeability, fluid accumulation, and the migration of a changing population of inflammatory cells into the inflamed area. The clinical manifestations of inflammation include swelling (edema), increased local temperature, erythema, and pain. The inflammatory response can be triggered by any of a number of causative factors, including certain bacteria, radiation, hypersensitivity to chemical agents, arthritis-like conditions, and the like. The inflammatory response is generally believed to be a primary defense mechanism in the body, but, unchecked, can become excessive and can result in functional impairment.

The use of non-steroidal anti-inflammatory, antipyretic and analgesic drugs, especially the salicylates, which include aspirin and aspirin derivatives, to combat inflammation and attendant pain is accepted medical practice. The non-steroidals are commonly employed to relieve pain and inflammation associated with, for example, bursitis, arthritis, and the like.

While « pain » is incapable of precise definition due to its basically subjective nature, it can generally be said that the term refers to feelings of distress or suffering caused by stimulation of specialized nerve endings. A great variety of drugs have been developed to reduce pain in man and other animals ; some directed to eliminating pain at its source, and others directed to blocking the assimilation of pain by the brain. Among the latter group of drugs that are designed to block the sensation of pain, are the analgesics, which generally relieve pain without causing unconsciousness. Analgesics can be further classified in two main categories : opioid analgesics, including morphine, codeine, levorphanol, and the morphine-like analgesics merperidine, and methadone ; and antipyretic analgesics, such as aspirin, phenacetin, acetaminophen, phenylbutazone, and indomethacin.

Although the precise pharmacological action of these analgesics is uncertain, there are certain effects which readily distinguish the opioid analgesics from the antipyretics. In particular, the antipyretics are weak analgesics, with much of their effect in the peripheral nervous system, so that behavioral changes do not usually occur. Generally, these analgesics relieve only somatic pain originating from muscles, joints, tendons and fasciae, and are ineffective against deep visceral pain. However, the opioid analgesics are quite effective against all types of pain, with broad-based action in the central nervous system. Aside from potent analgesia, the opioids, also known as narcotics, often produce effects on mood and other behavioral changes. Perhaps the most notable side effect of the opioid analgesics is the fact that their repeated use is associated with tolerance, as well as psychic and physical dependence.

It has been recently discovered that capsaicin, a natural product of certain species of the genus Capsicium, induces analgesia. Capsaicin (8-methyl-N-vanillyl-6-nonen-amide) and « synthetic » capsaicin (N-vanillylnonamide) are disclosed as analgesics in U.S. Patent 4 313 958, LaHann, issued February 2, 1982. Analgesic activity of capsaicin has also been discussed in the chemical and medical literature, including Yaksh, et al, Science, 206, pp 481-483 (1979) ; Jancso, et al, Naunyn-Schmiedeberg's Arch. Pharmacol., Vol. 311, pp 285-288 (1980) and Holzer et al, Eur. J. Pharm. Vol. 58, pp 511-514 (1979). U.S. Patent 4 238 505, Nelson, issued December 9, 1980, discloses 3-hydroxyacetanilide for use in producing analgesia in animals. European Patent Application 0 089 710, LaHann, et al, published September 28, 1983, describes hydroxyphenylacetamides with analgesic and anti-irritant activity. Similarly, analgesic and anti-irritant activity is disclosed for N-vanillyl sulfonamides in U.S. Patent 4 401 663, Buckwalter, et al, issued August 30, 1983 ; N-vanillylureas in European Patent Application 0 068 590, Buckwalter, et al, published January 5, 1983 ; N-(substituted phenyl) methyl alkynamides in U.S. Patent Application Serial No. 514, 204, Janusz, et al, filed July 14, 1983 ; methylene substituted N-(substituted phenyl) methyl alkanamides in U.S. Patent Application Serial No. 514 205, Janusz, et al, filed July 14, 1983 ; N-(substituted phenyl) methyl-cis-monounsaturated alkenamides in U.S. Patent Application Serial No. 514 206, LaHann, et al, filed July 14, 1983 ; and N-(substituted phenyl) methyl diunsaturated amides in U.S. Patent Application Serial No. 514 207, LaHann, et al, filed July 14, 1983.

It has now been discovered that certain trienamides have anti-inflammatory and analgesic activity in humans and lower animals. Some of these trienamides have analgesic potency far greater than that of aspirin and comparable to that of the opioids, but do not exhibit undesirable narcotic side effects such as tolerance and physical dependence. These trienamides are also less toxic than capsaicin.

### Summary of the invention

The present invention provides compounds useful for reducing inflammation and producing

analgesia in humans and lower animals, of the formula :

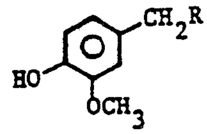

wherein R is straight or branched chain tri-unsaturated fatty acid amide having from 14 to 24 carbon atoms ; and pharmaceutically-acceptable salts thereof.

This invention also provides pharmaceutical compositions comprising a safe and effective amount of these compounds, or mixtures thereof, and a pharmaceutically-acceptable carrier.

Description of the invention

The compositions of this invention incorporate certain N-vanillyl trienamides, or pharmaceutically-acceptable salts thereof, of the formula :

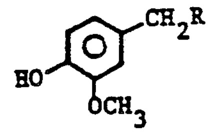

wherein R is a straight or branched chain tri-unsaturated fatty acid amide, preferably a cis, straight-chain tri-unsaturated fatty acid amide, having from 14 to 24 carbon atoms, and preferably from 18 to 20 carbon atoms.

Preferred trienamides include those wherein R is derived from such cis-triunsaturated fatty acids as 11Z, 14Z, 17Z-eicosatrienoic acid, γ-linolenic acid, and linolenic acid. Particularly preferred trienamides include N-vanillyl-9Z, 12Z, 15Z-octadecatrienamide (N-vanillyl-linolenamide), N-vanillyl-6Z, 9Z, 12Z-octadecatrienamide (N-vanillyl-γ-linolenamide), and N-vanillyl-11Z, 14Z, 17Z-eicosatrienamide. The most preferred trienamide for anti-inflammatory activity is N-vanillyl-6Z, 9Z, 12Z-octadecatrienamide. The most preferred trienamide for analgesic activity is N-vanillyl-11Z, 14Z, 17Z-eicosatrienamide, which appears to have analgesic activity comparable to that of the opioids, but does not exhibit undesirable narcotic side effects. The other trienamides tested exhibit a much weaker analgesic effect, possibly comparable to that of aspirin. Preferred pharmaceutically-acceptable trienamide salts include the sodium, potassium, calcium, magnesium, and ammonium salts.

The trienamides described herein can be readily prepared by the following general synthetic scheme :

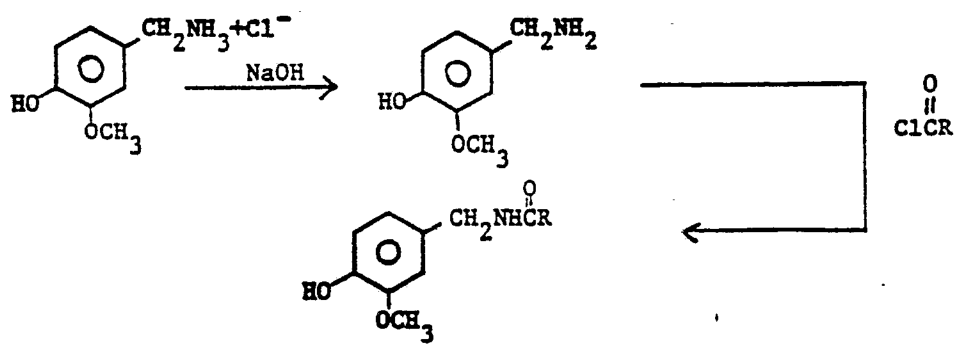


The fatty acids used in the synthesis of preferred tri-enamides are commercially-available.

Compositions

The compositions of the present invention comprise :

(a) a safe and effective amount of a trienamide as defined herein ; and

(b) a pharmaceutically-acceptable carrier.

A safe and effective amount of trienamide is that amount which provides anti-inflammatory activity and analgesia, thereby alleviating or preventing the inflammation or pain being treated at a reasonable benefit/risk ratio, as is intended with any medical treatment. Obviously, the amount of trienamide used will vary with such factors as the particular condition that is being treated, the severity of the condition, the duration of the treatment, the physical condition of the patient, the nature of concurrent therapy (if any),

the route of administration, the specific formulation and carrier employed, and the solubility and concentration of trienamide therein.

Depending upon the particular route of administration, and compatibility with the active chosen, a variety of pharmaceutically-acceptable carriers, well-known in the art, may be used. These include solid or liquid fillers, diluents, hydrotropes, excipients, surface-active agents, and encapsulating substances. The amount of the carrier employed in conjunction with the trienamide is sufficient to provide a practical quantity of material per unit dose.

Pharmaceutically-acceptable carriers for systemic administration that may be incorpored into the compositions of this invention, include sugars, starches, cellulose and its derivatives, malt, gelatin, talc, calcium sulfate, vegetable oils, synthetic oils, polyols, alginic acid, phosphate buffer solutions, emulsifiers, isotonic saline, and pyrogen-free water. Specific pharmaceutically-acceptable carriers are described in the following U.S. Patents and European Patent Applications, all incorporated by reference herein : U.S. Patent 4 401 663, Buckwalter, et al, issued August 30, 1983 ; European Patent Application 0 089 710, LaHann, et al, published September 28, 1983 ; and European Patent Application 0 068 592, Buckwalter, et al, published January 5, 1983. Preferred carriers for parenteral administration include propylene glycol, ethyl oleate, pyrrolidone, ethanol, and vegetable oils. Preferably, the pharmaceutically-acceptable carrier, in compositions for parenteral administration, comprises at least about 90 % by weight of the total composition.

Various oral dosage forms can be used, including such solid forms as tablets, capsules, granules and bulk powders. These oral forms comprise a safe and effective amount, usually at least about 5 %, and preferably from about 25 % to about 50 % of the trienamide. Tablets can be compressed, tablet triturates, enteric-coated, sugar-coated, film-coated or multiple compressed, containing suitable blinders, lubricants, diluents, disintegrating agents, coloring agents, flavoring agents, preservatives, flow-inducing agents, and melting agents. Liquid oral dosage forms include aqueous solutions, emulsions, suspensions, solutions and/or suspensions reconstituted from non-effervescent granules and effervescent preparations reconstituted from effervescent granules, containing suitable solvents, preservatives, emulsifying agents, suspending agents, diluents, sweeteners, melting agents, coloring agents, and flavoring agents. Preferred carriers for oral administration include gelatin, propylene glycol, ethyl oleate, cottonseed oil and sesame oil. Specific examples of pharmaceutically-acceptable carriers and excipients that may be used to formulate oral dosage forms, which may be used in formulating oral dosage forms containing trienamides, are described in U.S. Patent 3 903 297, Robert, issued September 2, 1975, incorporated by reference herein. Techniques and compositions for making solid oral dosage forms are described in Marshall, « Solid Oral Dosage Forms, » Modern Pharmaceutics, Vol. 7, (Banker and Rhodes, editors), 359-427 (1979), incorporated by reference herein.

The compositions of the present invention can also be administered topically to a biologic subject, i.e., by the direct laying on or spreading of the composition on epidermal or epithelial tissue. Such compositions include lotions, creams, solutions, gels and solids. These topical compositions comprise a safe and effective amount, usually at least about 0.5 %, and preferably from about 1 % to about 5 %, of the trienamide. Suitable carriers for topical administration of the trienamide preferably remain in place on the skin as a continuous film and resist being washed off easily by perspiration or by immersion in water. Generally, the carrier is either organic in nature or an aqueous emulsion and capable of having the trienamide dispersed or dissolved therein. The carrier may include pharmaceutically-acceptable emollients, coloring agents, fragrances, emulsifiers, thickening agents, and solvents.

Specific systemic and topical formulations useful in this invention are described in the following U.S. Patents and European Patent Applications, all incorporated by reference herein : U.S. Patent No. 4 401 663, Buckwalter, et al, issued August 30, 1983 ; and European Patent Application 0 089 710 ; LaHann, et al, published September 28, 1983 ; European Patent Application 0 068 590, Buckwalter, et al, published January 5, 1983 ; and European Patent Application 0 068 592, Buckwalter, et al, published January 5, 1983. Topical vehicles, useful herein, are disclosed in the following U.S. Patent Applications, incorporated by reference herein : « Improved Penetrating Topical Pharmaceutical Compositions Combining 1-dodecylazacycloheptan-2-one », Serial No. 506 275, Cooper, filed June 21, 1983 ; and « Penetrating Topical Pharmaceutical Compositions Containing N-(2-hydroxyethyl)-pyrrolidone », Serial No. 506 273, Cooper, filed June 21, 1983. Additional formulations, useful for parenteral, oral, and topical administration of trienamides, are disclosed in the following U.S. Patent Applications all incorporated by reference herein : « Compositions Useful for Producing Analgesia », Serial No. 514 206, LaHann and Buckwalter, filed July 14, 1983 ; « Novel Compounds and Compositions Useful for Producing Analgesia », Serial No. 514 207, LaHann, Janusz, and Buckwalter, filed July 14, 1983 ; « Novel Compounds Useful for Producing Analgesia », Serial No. 514 204 Janusz and LaHann, filed July 14, 1983 ; and « Novel Compounds and Compositions Useful for Producing Analgesia », Serial No. 514 205 Janusz, Buckwalter and LaHann, filed July 14, 1983.

Methods for Producing Anti-Inflammatory Activity And Analgesia

Anti-inflammatory activity and analgesia in humans or lower animals can be produced by administering, to the human or lower animal, a safe and effective amount, usually from about 1 mg to about 3600 mg

per day, preferably from about 200 mg to about 2000 mg per day, of a trienamide described herein. While dosages higher than the foregoing are effective to reduce inflammation and produce analgesia, care must be taken in some individuals to prevent adverse side effects. The trienamides and compositions of this invention can be used to treat and prevent pain, to provide analgesia, and to reduce inflammation in various disorders at the deeper structures, muscles, tendons, bursa and joints associated with disease and trauma, and in various other conditions in which non-steroidal anti-inflammatory, antipyretic and analgesic drugs such as aspirin and opioids such as morphine have heretofore been used to alleviate pain and discomfort and reduce inflammation.

The trienamides and compositions of the instant invention can be administered topically or systemically. Systemic application includes any method of introducing the trienamide compound into the tissues of the body, e.g., intrathecal, epidural, intramuscular, transdermal, intravenous, intraperitoneal, subcutaneous, sublingual, and oral administration.

A preferred method of parenteral administration is through intramuscular injection. As is known and practiced in the art, all formulations for parenteral administration must be sterile. For mammals, especially humans, individual doses of from about 0.025 mg/kg to about 6.0 mg/kg of trienamide are acceptable. Thus, a human weighing approximately 70 kg could be given an individual dose of from about 2 mg to about 400 mg of trienamide. Individual doses of from about 1.0 mg/kg to about 3.0 mg/kg are preferred. Although frequency of administration will be determined by the duration of activity of the particular trienamide administered, which is variable, the trienamides are generally long-acting, and in some cases it may be possible to obtain effective relief by administering the composition as infrequently as once every 2-3 days.

A preferred method of systemic application of the trienamides is through oral administration. For mammals, especially humans, individual doses of from about 0.015 mg/kg to about 20 mg/kg of trienamide are acceptable. Thus, a human weighing approximately 70 kg could be given an individual dose of from about 1 mg to about 1500 mg of trienamide. Individual doses of from about 1.0 mg/kg to about 8.0 mg/kg are especially preferred.

Topical administration can be used to reduce inflammation and produce local or systemic analgesia, through directly laying on or spreading a safe and effective amount of the trienamides, or composition containing a trienamide, on epidermal or epithelial tissue, including outer skin and oral, gingival, and nasal tissue. The amount of the pharmaceutical composition to be topically administered may vary from about 1 $mg/cm^2$ to 5 $mg/cm^2$, and if a patch is worn over the affected area possibly higher, depending upon such factors as the sensitivity, type and location of tissue to be treated, the composition and carrier (if any) to be administered, and the particular trienamide to be administered as well as the particular disorder to be treated and the extent to which systemic (as distinguished from local) effects are desired. The extent of systemic analgesia also depends upon such factors as the amount of trienamide, the area of tissue to be covered, and the ability of the trienamide composition to prenetrate the skin tissues.

The following Examples illustrate the compounds and compositions of the present invention.

Example I

N-vanillyl-6Z, 9Z, 12Z-octadecatrienamide was synthesized by the following method :

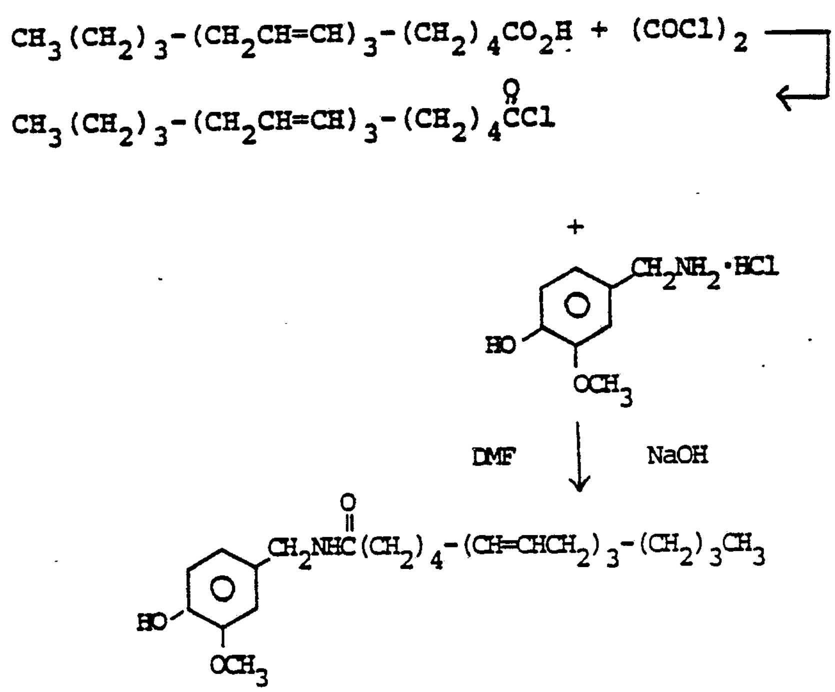

Specifically, 3.17 g (2.18 ml) of oxalyl chloride and 5 g of γ-linolenic acid were added to 30 ml of dry methylene chloride, and the mixture was refluxed for approximately 90 minutes, until gas evolution ceased. Excess oxalyl chloride and the solvent were evaporated. 3.41 g of vanillylamine hydrochloride was suspended in 60 ml of N,N-dimethylformamide (DMF). 7.2 ml of 5N NaOH was added, and the mixture was stirred at room temperature for 10 minutes, then cooled to 0 °C. 40 ml of ether was added to the γ-linolenic acid chloride, and the resulting solution was added dropwise to the vanillylamine mixture over a 20 minute time period. The mixture was allowed to warm to room temperature and stored overnight. The next morning, the mixture was poured into 600 ml of water, and extracted with 250 ml of ether. This process was repeated 3 times. Extracts were combined and washed with 1N HCl, saturated $NaHCO_3$, and brine, dried over $MgSO_4$, and evaporated. 7.2 g of crude N-vanillyl-6Z, 9Z, 12Z-octadecatrienamide was obtained. The crude product was flash chromatographed with 33 % EtOAC/hexane. 5.45 g of analytically pure product was obtained.

In the above example, N-vanillyl-9Z, 12Z, 15Z-octadecatrienamide was made by substituting the appropriate linolenic acid in the above synthesis.

Example II

N-vanillyl-11Z, 14Z, 17Z-eicosatrienamide was synthesized by the following method :

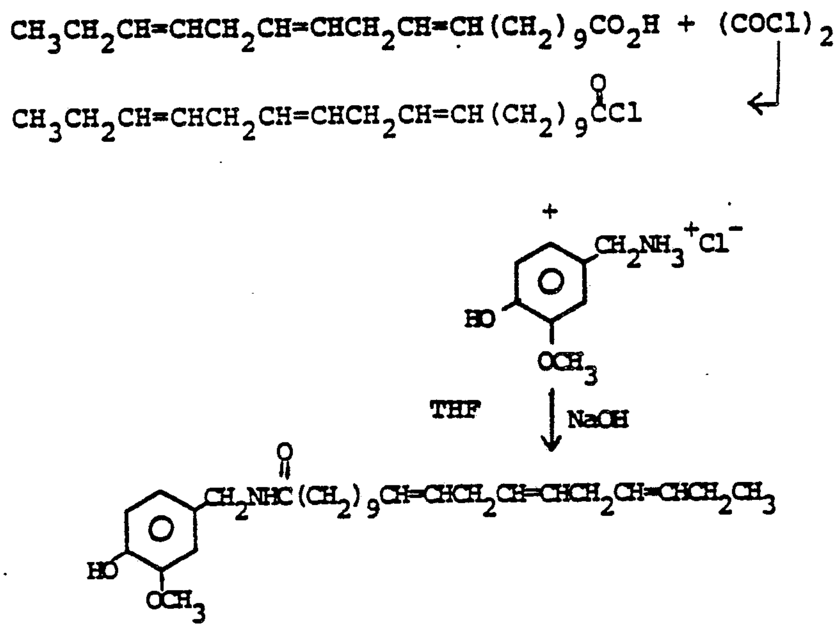


Specifically, 1.2 ml of oxalyl chloride was added to 4.0 g of eicosatrienoic acid in 10 ml of chloroform and stirred at room temperature for 60 minutes, then warmed to 50 °C for 20 minutes. Excess solvent and oxalyl chloride were evaporated. 5.76 ml of 5N NaOH was then added to 2.71 g of vanillylamine hydrochloride in 50 ml of tetrahydrofuran (THF) and stirred for 15 minutes. 25 ml of ether was added to the acid chloride and the resulting solution was added dropwise to the vanillylamine mixture over 15 minutes. The reaction was stirred at room temperature and then refrigerated overnight. The following morning, the solvent was evaporated and the residue partitioned between 50 ml ethyl ether and 50 ml water. The organic phase extract was washed with 1N HCl, saturated $NaHCO_3$, $H_2O$, and brine, and then dried over $MgSO_4$, filtered, and evaporated. 6.0 g of crude N-vanillyl-11Z, 14Z, 17Z-eicosatrienamide was obtained. The crude product was flash chromatographed with 40 % EtoAc/hexane. 5.0 g of analytically pure product was obtained.

Example III

A composition for parenternal administration is prepared by combining the following ingredients :

| | |
|---|---|
| N-vanillyl-9Z, 12Z, 15Z-octadecatrienamide | 300 g |
| Ethyl oleate | 980 ml |
| Benzyl alcohol | 20 ml |

The octadecatrienamide is dissolved in the solution combining ethyl oleate and benzyl alcohol, sealed into airtight 5 ml ampoules, and sterilized by autoclaving. Injection of 1.5 ml of the contents of one

of these ampoules intramuscularly into a 65 kg human produces analgesia and reduces inflammation.
A substantially similar reduction of inflammation, but a weaker analgesic effect, is obtained when N-vanillyl-9Z, 12Z, 15Z-octadecatrienamide is replaced with N-vanillyl-6Z, 9Z, 12Z-octadecatrienamide or N-vanillyl-11Z, 14Z, 17Z-eicosatrienamide.

Example IV

A composition for oral administration is prepared by combining the following ingredients :

| | |
|---|---|
| N-vanillyl-9Z, 12Z, 15Z-octadecatrienamide | 1.10 kg |
| Sesame oil | 3.25 liters |

The octadecatrienamide is dissolved in the sesame oil with the aid of sonication and is packaged in soft gelatin capsules using methods known in the art. Two of the resulting capsules, each containing 225 mg of the composition, are administered to a 60 kg human, producing analgesia and reducing inflammation.
A substantially similar reduction of inflammation, but a weaker analgesic effect, is obtained when the N-vanillyl-9Z, 12Z, 15Z-octadecatrienamide is replaced with N-vanillyl-6Z, 9Z, 12Z-octadecatrienamide or N-vanillyl-11Z, 14Z, 17Z-eicosatrienamide.

Example V

A composition for oral administration is prepared by combining the following ingredients :

| | |
|---|---|
| N-vanillyl-9Z, 12Z, 15Z-octadecatrienamide | 250 g |
| Propylene glycol | 1800 ml |
| Ethyl alcohol | 175 ml |
| Distilled water | 75 ml |
| Artificial Strawberry flavor | 10 ml |
| FD&C Red #40 | 0.2 g |

The above ingredients are combined to produce a syrup and are packaged under sterile conditions in 6 oz. bottles. One teaspoon of this formulation is administered to a 70 kg adult human, reducing inflammation and producing analgesia.
A substantially similar reduction of inflammation, but a weaker analgesic effect, is obtained when the N-vanillyl-9Z, 12Z, 15Z-octadecatrienamide is replaced with N-vanillyl-6Z, 9Z, 12Z-octadecatrienamide or N-vanillyl-11Z, 14Z, 17Z-eicosatrienamide.

Example VI

A composition for topical administration is prepared by combining the following ingredients :

| | |
|---|---|
| N-vanillyl-9Z, 12Z, 15Z-octadecatrienamide | 4 g |
| Propylene glycol | 100 ml |
| Ethyl alcohol | 100 ml |

The octadecatrienamide is melted to a liquid with slight warming and combined with the other ingredients. Application of 0.4 ml of the resulting liquid to a 80 $cm^2$ portion of the forearm of a 60 kg human reduces inflammation and produces analgesia.
A substantially similar reduction of inflammation, but a weaker analgesic effect, is obtained when the N-vanillyl-9Z, 12Z, 15Z-octadecatrienamide is replaced with N-vanillyl-6Z, 9Z, 12Z-octadecatrienamide or N-vanillyl-11Z, 14Z, 17Z-eicosatrienamide.

Effectiveness in Reducing Inflammation and Providing Analgesia

Example VII

Two trienamide compositions were tested for anti-inflammatory activity using the croton oil-induced mouse ear inflammation test.
Adult male Cox ICR mice, 20-30 g, were treated on the left ear at 20-28 hours prior to sacrifice and a second time 5-6 hours prior to sacrifice with 25 μl of a 1 % ethanolic solution of the test compound. Four hours prior to sacrifice both ears were treated with 25 μl of a 2 % solution of croton oil in acetone. Each animal was then placed in individual cages and given food and water ad lib. Animals were sacrificed by cervical dislocation and both ears removed. From these ears, 0.38 $cm^2$ punch biopsies were taken from the central portion and each biopsy weighed on a Cahn electrobalance.
For each test substance, a group of 10 animals was used. Control groups either had both ears treated

with croton oil or just the right ear. It was experimentally determined that a value of 11.0 mg could be assumed for a punch biopsy from a normal untreated ear and still be within the experimental error of the test. Therefore, for the calculation of percent inhibition, a value of 11.0 mg was used.

$$\frac{\text{Weight Right Ear} - \text{Weight Left Ear}}{\text{Weight Right Ear} - \text{Weight Control Ear (11.0 mg)}} \times 100$$

This calculation is valid only when no systemic effects are noted as evidenced by comparison of right ears of treated and control groups.

Statistical significance at the 95 % confidence level was determined by the paired t test.

| Compound | %Inhibition |
|---|---|
| N-vanillyl-9Z, 12Z, 15Z-octadecatrienamide | 78.5 ± 31.9 |
| N-vanillyl-6Z, 9Z, 12Z-octadecatrienamide | 100.7 ± 11.9 |

These results show that the trienamide compositions tested do in fact have statistically significant anti-inflammatory activity.

## Example VIII

Three trienamide compositions were tested for analgesic and anti-inflammatory activity using the phenylquinone writhing assay.

Groups of eight male mice weighing between approximately 25 and 30 g were dosed orally by gavage with the composition to be tested. Identical groups of mice were dosed with control compositions. Three hours after this initial administration, the mice were injected intraperitoneally with a 0.2 % solution of phenylbenzoquinone in aqueous ethanol. The ability of the analgesic compositions tested to relieve the discomfort induced was measured by counting the number of abdominal contractions, or « writhes », occurring in each mouse during a 10 minute period beginning 10 minutes after injection of the phenylbenzoquinone solution.

| Compound | Writhes/10 minutes |
|---|---|
| N-vanillyl-9Z, 12Z, 15Z-octadecatrienamide | 0.4 ± 0.3 |
| N-vanillyl-6Z, 9Z, 12Z-octadecatrienamide | 0.0 ± 0.0 |
| N-vanillyl-11Z, 14Z, 17Z-eicosatrienamide | 0.4 ± 0.3 |
| Vehicle Control | 17.4 ± 1.7 |

These results show that the trienamide compositions tested exhibit analgesic/anti-inflammatory activity.

### Rodent Hot Plate Test

The degree of thermal analgesia obtained was determined using the « rodent hot plate test » (RHP). The RHP system is designed to detect and evaluate agents which elevate the threshold for the perception of pain. Classically, this method has been utilized primarily to evaluate opioid (narcotic) analgesic agents, such as morphine. Unless administered in toxic quantities, antipyretic analgesics, such as aspirin or acetaminophen, exhibit little or no activity in the RHP system.

Groups of 10 male CF-1 mice or 10 male Sprague-Dawley rats were used to evaluate each composition. The test procedure consisted of placing a particular rodent on a surface heated to 55 °C and observing its behavior. The point in time at which the rodent either rapidly fanned one of its rear paws or licked any one of its paws was noted, and the total elapsed time from the first contact with the heated surface was determined (« response time »). If the response time for a particular rodent reached sixty seconds, the rodent was removed from the hot plate so as to prevent organic damage, and the response time recorded as sixty seconds. Hence, the maximum measurable response time for any particular composition was sixty seconds.

## Example IX

An analgesic composition for oral administration was made with the following ingredients :

| | |
|---|---|
| N-vanillyllinolenamide (VL) | 400 mg |
| Propylene glycol | 5 ml |

The n-vanillyllinolenamide was dissolved in the propylene glycol with the aid of sonication. Groups of 10 male Sprague-Dawley rats (100-250 g) were dosed orally by gavage with either the vehicle (VC) alone or

the analgesic composition (VL).

The analgesic activity was then measured using the Rodent Hot Plate Test described above. Rodents were considered analgesic if their post-dose latency time was greater than the sum of their individual pre-dose latency time plus 3 times the standard deviation of the group's pre-dose latency times. The percent of rodents demonstrating analgesia was measured at 1, 2, and 3 hours post-dose.

Experiment A

| Treatment | % Animals Analgesic | | |
|---|---|---|---|
| | 1 hr. | 2 hrs. | 3 hrs. post-dose |
| VL-200 mg/kg | 80 | 40 | 30 |
| VC-2.5 ml/kg | 20 | 10 | 0 |

Experiment B

| Treatment | % Animals Analgesic | | |
|---|---|---|---|
| | 1 hr. | 2 hrs. | 3 hrs. post-dose |
| VL-200 mg/kg | 50 | 60 | 20 |
| VC-2.5 ml/kg | 0 | 20 | 0 |

Experiment C

| Treatment | % Animals Analgesic | | |
|---|---|---|---|
| | 1 hr. | 2 hrs. | 3 hrs. post-dose |
| VL-288 mg/kg | 50 | 10 | 0 |
| VC-3.6 ml/kg | 10 | 0 | 0 |

Example X

An analgesic composition for oral administration was made with the following ingredients :

| | |
|---|---|
| N-vanillyllinolenamide | 400 mg |
| Sesame oil | 5 ml |

The N-vanillyllinolenamide (VL) was dissolved in the sesame oil with the aid of sonication. Groups of 10 male Sprague-Dawley rats (100-250 g) were dosed orally by gavage with either the vehicle alone (VC) or the analgesic composition (VL).

The analgesic activity was then measured using the Rodent Hot Plate Test described above. The percent of rodents demonstrating analgesia was measured at 1, 2, and 3 hours post-dose.

| Treatment | % Animals Analgesic | | |
|---|---|---|---|
| | 1 hr. | 2 hrs. | 3 hrs. post-dose |
| VL-200 mg/kg | 70 | 30 | 10 |
| VC-2.5 ml/kg | 0 | 20 | 0 |

## Claims

1. Trienamide compounds, and pharmaceutically-acceptable salts thereof, of the formula :

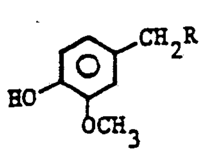

wherein R is a straight or branched chain tri-unsaturated fatty acid amide having from 14 to 24 carbon atoms.

2. Trienamide compounds, and pharmaceutically-acceptable salts thereof, according to Claim 1, wherein R is a straight or branched chain tri-unsaturated fatty acid amide having from 18 to 20 carbon atoms.

3. Trienamide compounds, and pharmaceutically-acceptable salts thereof, according to Claim 1, wherein R is a straight chain tri-unsaturated fatty acid amide.

4. Trienamide compounds, and pharmaceutically-acceptable salts thereof, according to Claim 3, wherein R is a straight chain tri-unsaturated fatty acid amide having from 18 to 20 carbon atoms.

5. The trienamide compound, according to Claim 4, wherein said trienamide is N-vanillyl-9Z, 12Z, 15Z-octadecatrienamide and pharmaceutically-acceptable salts thereof.

6. The trienamide compound, according to Claim 4, wherein said trienamide is N-vanillyl-6Z, 9Z, 12Z-octadecatrienamide and pharmaceutically-acceptable salts thereof.

7. The trienamide compound, according to Claim 4, wherein said trienamide is N-vanillyl-11Z, 14Z, 17Z-eicosatrienamide and pharmaceutically-acceptable salts thereof.

8. A composition for reducing inflammation and producing analgesia in humans or lower animals comprising :

(a) a safe and effective amount of a trienamide compound of the formula

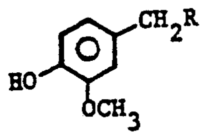

wherein R is a straight or branched chain tri-unsaturated fatty acid amide having from 14 to 24 carbon atoms, or a pharmaceutically-acceptable salt thereof, or mixtures thereof ; and

(b) a pharmaceutically-acceptable carrier.

9. A composition, according to Claim 8, wherein R is a straight or branched chain tri-unsaturated fatty acid amide having from 18 to 20 carbon atoms.

10. A composition, according to Claim 8, wherein R is a straight chain tri-unsaturated fatty acid amide.

11. A composition, according to Claim 10, wherein R is a straight chain tri-unsaturated fatty acid amide having from 18 to 20 carbon atoms.

12. A composition, according to Claim 11, wherein said trienamide compound is N-vanillyl-9Z, 12Z, 15Z-octadecatrienamide.

13. A composition, according to Claim 11, wherein said trienamide compound is N-vanillyl-6Z, 9Z, 12Z-octadecatrienamide.

14. A composition, according to Claim 11, particularly useful for reducing inflammation, wherein said trienamide compound is N-vanillyl-11Z, 14Z, 17Z-eicosatrienamide.

15. A composition, according to Claim 8, for parenteral administration, comprising at least about 90 %, by weight, of said pharmaceutically-acceptable carrier.

16. A composition, according to Claim 8, for oral administration, comprising from about 25 % to about 50 %, by weight, of said trienamide.

17. A composition, according to Claim 8, for topical administration, comprising from about 1 % to about 5 %, by weight, of said trienamide.

**Patentansprüche**

1. Trienamidverbindungen der Formel

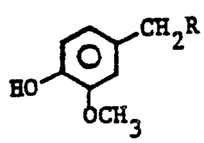

worin R ein geradkettiges oder verzweigtkettiges, dreifach ungesättigtes Fettsäureamid mit 14 bis 24 Kohlenstoffatomen ist, und deren pharmazeutisch annehmbare Salze.

2. Trienamidverbindungen und pharmazeutisch annehmbare Salze davon nach Anspruch 1, wobei R ein geradkettiges oder verzweigtkettiges, dreifach ungesättigtes Fettsäureamid mit 18 bis 20 Kohlenstoffatomen ist.

3. Trienamidverbindungen und pharmazeutisch annehmbare Salze davon nach Anspruch 1, wobei R ein geradkettiges, dreifach ungesättigtes Fettsäureamid ist.

4. Trienamidverbindungen und pharmazeutisch annehmbare Salze davon nach Anspruch 3, wobei R ein geradkettiges, dreifach ungesättigtes Fettsäureamid mit 18 bis 20 Kohlenstoffatomen ist.

5. Die Trienamidverbindung nach Anspruch 4, wobei das genannte Trienamid N-Vanillyl-9Z, 12Z, 15Z-octadecatrienamid ist, und pharmazeutisch annehmbare Salze davon.

6. Die Trienamidverbindung nach Anspruch 4, wobei das genannte Trienamid N-Vanillyl-6Z, 9Z, 12Z-octadecatrienamid ist, und pharmazeutisch annehmbare Salze davon.

7. Die Trienamidverbindung nach Anspruch 4, wobei das genannte Trienamid N-Vanillyl-11Z, 14Z, 17Z-eicosatrienamid ist, und pharmazeutisch annehmbare Salze davon.

8. Eine Zusammensetzung zum Verringern von Entzündung und zum Hervorrufen von Analgesie bei Menschen oder niedrigeren Tieren, enthaltend :

a) eine sichere und wirksame Menge einer Trienamidverbindung der Formel

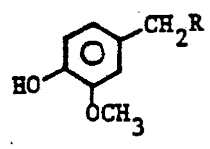

worin R ein geradkettiges oder verzweigtkettiges, dreifach ungesättigtes Fettsäureamid mit 14 bis 24 Kohlenstoffatomen ist, oder eines pharmazeutisch annehmbaren Salzes davon, oder von Gemischen davon ; und

b) einen pharmazeutisch annehmbaren Träger.

9. Eine Zusammensetzung nach Anspruch 8, wobei R ein geradkettiges oder verzweigtkettiges, dreifach ungesättigtes Fettsäureamid mit 18 bis 20 Kohlenstoffatomen ist.

10. Eine Zusammensetzung nach Anspruch 8, wobei R ein geradkettiges, dreifach ungesättigtes Fettsäureamid ist.

11. Eine Zusammensetzung nach Anspruch 10, wobei R ein geradkettiges, dreifach ungesättigtes Fettsäureamid mit 18 bis 20 Kohlenstoffatomen ist.

12. Eine Zusammensetzung nach Anspruch 11, wobei die genannte Trienamidverbindung N-Vanillyl-9Z, 12Z, 15Z-octadecatrienamid ist.

13. Eine Zusammensetzung nach Anspruch 11, wobei die genannte Trienamidverbindung N-Vanillyl-6Z, 9Z, 12Z-octadecatrienamid ist.

14. Eine Zusammensetzung nach Anspruch 11, die insbesondere zur Verringerung von Entzündung nützlich ist, wobei die genannte Trienamidverbindung N-Vanillyl-11Z, 14Z, 17Z-eicosatrienamid ist.

15. Eine Zusammensetzung nach Anspruch 8 zur parenteralen Verabreichung, enthaltend wenigstens etwa 90 Gew.-% des genannten pharmazeutisch annehmbaren Trägers.

16. Eine Zusammensetzung nach Anspruch 8 zur oralen Verabreichung, enthaltend etwa 25 Gew.-% bis etwa 50 Gew.-% des genannten Trienamids.

17. Eine Zusammensetzung nach Anspruch 8 zur topischen Verabreichung, enthaltend etwa 1 Gew.-% bis etwa 5 Gew.-% des genannten Trienamids.

**Revendications**

1. Composés triénamides, et leurs sels pharmaceutiquement acceptables, de formule :

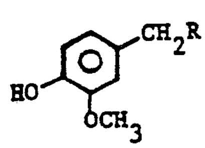

dans laquelle R est un amide d'acide gras tri-insaturé à chaîne droite ou à chaîne ramifiée ayant 14 à 24 atomes de carbone.

2. Composés triénamides, et leurs sels pharmaceutiquement acceptables, selon la revendication 1, dans lesquels R est un amide d'acide gras tri-insaturé à chaîne droite ou à chaîne ramifiée ayant 18 à 20 atomes de carbone.

3. Composés triénamides, et leurs sels pharmaceutiquement acceptables, selon la revendication 1, dans lesquels R est un amide d'acide gras tri-insaturé à chaîne droite.

4. Composés triénamides, et leurs sels pharmaceutiquement acceptables, selon la revendication 3, dans lesquels R est un amide d'acide gras tri-insaturé à chaîne droite ayant de 18 à 20 atomes de carbone.

5. Composé triénamide selon la revendication 4, dans lequel le triénamide est le N-vanillyl-9Z, 12Z, 15Z-octadécatriénamide et ses sels pharmaceutiquement acceptables.

6. Composé triénamide selon la revendication 4, dans lequel le triénamide est le N-vanillyl-6Z, 9Z, 12Z-octadécatriénamide et sels pharmaceutiquement acceptables.

7. Composé triénamide selon la revendication 4, dans lequel le triénamide est le N-vanillyl-11Z, 14Z, 17Z-eicosatriénamide et ses sels pharmaceutiquement acceptables.

8. Composition pour réduire l'inflammation et produire l'analgésie chez l'homme ou les animaux inférieurs, comprenant :

a) une quantité inoffensive et efficace d'un composé triénamide de formule

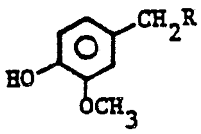

dans laquelle R est un amide d'acide gras tri-insaturé à chaîne droite ou à chaîne ramifiée ayant 14 à 24 atomes de carbone, ou un de ses sels pharmaceutiquement acceptables, ou des mélanges de ceux-ci ; et

b) un véhicule pharmaceutiquement acceptable.

9. Composition selon la revendication 8, dans laquelle R est un amide d'acide gras tri-insaturé à chaîne droite ou à chaîne ramifiée ayant 18 à 20 atomes de carbone.

10. Composition selon la revendication 8, dans laquelle R est un amide d'acide gras tri-insaturé à chaîne droite.

11. Composition selon la revendication 10, dans laquelle R est un amide d'acide gras tri-insaturé à chaîne droite ayant de 18 à 20 atomes de carbone.

12. Composition selon la revendication 11, dans laquelle le triénamide est le N-vanillyl-9Z, 12Z, 15Z-octadécatriénamide.

13. Composition selon la revendication 11, dans laquelle le triénamide est le N-vanillyl-6Z, 9Z, 12Z-octadécatriénamide.

14. Composition selon la revendication 11, particulièrement utile pour réduire l'inflammation, dans laquelle le triénamide est le N-vanillyl-11Z, 14Z, 17Z-eicosatriénamide.

15. Composition selon la revendication 8, destinée à l'administration parentérale, comprenant au moins environ 90 % en poids du véhicule pharmaceutiquement acceptable.

16. Composition selon la revendication 8, destinée à l'administration orale, comprenant environ 25 % à environ 50 % en poids du triénamide.

17. Composition selon la revendication 8, destinée à l'administration topique, comprenant environ 1 % à environ 5 % en poids du triénamide.